# EUROPEAN PATENT SPECIFICATION

(11) **EP 3 812 038 B1**
(45) Date of publication and mention of the grant of the patent: **01.11.2023**
(21) Application number: 18908060.9
(22) Date of filing: 22.06.2018
(51) Int. Cl.: B01J 20/04, B01J 20/20, B01J 20/28, B01J 20/32, B01J 37/02

(54) **MULTILAYER ABSORBENT FILTERING ITEM; METHOD FOR OBTAINING SAID ITEM; AND USE OF SAME**
MEHRSCHICHTIGER ABSORBIERENDER FILTERARTIKEL, VERFAHREN ZUR HERSTELLUNG DES ARTIKELS UND VERWENDUNG DAVON
ARTICLE FILTRANT-ABSORBANT MULTICOUCHE ; PROCÉDÉ POUR OBTENIR LEDIT ARTICLE ; ET SON UTILISATION

(43) Date of publication of application: 28.04.2021
(73) Proprietor: Comercializadora Innvento S.A., 2520000 Viña del Mar (CL)
(72) Inventor: ARAYA LAZO, Igor Francisco, Viña del Mar (CL)
(74) Representative: Ibarra Garcia, Isabel
(86) International application number: PCT/CL2018/000022
(87) International publication number: WO 2019/165566

(56) References cited:
- EP-A1- 1 251 884
- WO-A1-2015/157873
- WO-A2-2014/140825
- US-A- 5 212 131
- US-A1- 2012 288 958
- US-A1- 2014 186 243
- LEBRERO, R. et al.: "Abatement of odorant compounds in one- and two-phase biotrickling filters under steady and transient conditions", Applied Microbiology and Biotechnology, vol. 97, no. 10, 2013, pages 4627-4638, XP055634130, ISSN: 0175-7598, DOI: 10.1007/s00253-012-4247-1
- XIE, Z. Z. et al.: "Adsorption properties of regenerative materials for removal of low concentration of toluene", Journal of the air & Waste Management Association, vol. 66, no. 12, 2016, pages 1224-1236, XP055634133, DOI: 10.1080/10962247.2016.1209257

## Description

The present invention relates to the development of an innovative technology referring to a multilayer adsorbent filtering item to eliminate offensive odours from bred farm animals, such as pigs and birds.

Specifically, a multilayer adsorbent filtering item that fulfils the function of continuously degrading compounds responsible for offensive odours is disclosed, offensive odours being understood as the odour generated by substances or industrial, commercial, or service activities which, while causes no harm to human health, constitutes a nuisance, such as those generated in an animal breeding centre due to the presence of volatile organic compounds (VOCs) (ammonia, hydrogen sulphide, methane, and carbon dioxide), which are the product of the decomposition of organic waste from animals. A method for obtaining said multilayer adsorbent filtering item is also disclosed.

### Description of the State of the Art

It is known that porous materials have covered numerous areas of research due to the applications that can be obtained with them. These materials are used as adsorbents, ion exchange systems, composite separations, and as catalysts or catalytic supports.

Although porous solids have a varied composition, they have in common the accessible space inside their structure. In other words, the aggregation of small particles of the solid results in the formation of pores within these grains and is defined as intra-particles or textural porosity. The diameter of the textural pores is directly related to the size of the grains forming these pores.

Different types of adsorbent structures and methods of application thereof have been disclosed in the prior art; specifically, document Cl200901573 relates to a method for impregnating a porous solid adsorbent, charcoal, with permanganate which comprises inertising the charcoal with a hydrophobic solvent by applying vacuum, contacting the inertised charcoal with an aqueous Na, K, or Li permanganate solution, removing excess liquid and drying the resulting solid; and porous solid adsorbent.

On the other hand, application WO 2015157873 discloses a method for impregnating a porous support with chemical(s), a porous support, and a system for impregnating said support. It aims to describe a method for impregnating a porous support with chemical(s), the optimum porous support to be used depending on the surface area, and a system specifically designed to carry out the impregnation method.

In addition, document WO1995013122 discloses a method for impregnating zeolite with a quaternary ammonium cation (QAC) and then coating the impregnated zeolite with permanganate (such as potassium permanganate) and for impregnating zeolite with permanganate and then coating the impregnated zeolite with QAC, and impregnated and coated zeolite crystals resulting from either method. Either coating acts as a protective agent for the impregnating substance within each zeolite crystal and allows regulated time release control of the impregnating substance, thus allowing a controlled rate of diffusion (or absorption) in applications in which coated impregnated zeolite is used to absorb contaminants from the air or water. The combinations of coated and uncoated zeolite crystals can be chosen to match specific environmental circumstances which can be calculated by analysis of the air or water to be treated. Coated and uncoated QAC-impregnated zeolite mixtures can be used to react with organic compounds such as benzene, toluene and xylene, uncoated permanganate-impregnated zeolite, and mixtures of coated and uncoated permanganate-impregnated zeolite can be used to react with hydrogen sulphide, acetone, ethylene glycols, formaldehyde, and other contaminants.

Methods for producing manganese dioxide-impregnated zeolite crystals, and for using said manganese dioxide-impregnated crystals to absorb contaminants from the fluid, are also disclosed.

In addition, document US8664153 relates to an adsorbent composition comprising: an activated carbon impregnated with at least one long chain saturated aliphatic hydrocarbon, a permanganate salt, and iron (III) oxide. Adsorbent compositions resulting from such methods are also disclosed in this document.

The present patent presents an original multilayer absorbent filtering item, with innovative technological improvements, which aims to mitigate odours from bred farm animals, such as pigs and birds.

Said multilayer absorbent filtering item is a multilayer adsorbent filtering item according to claim 2.

### Example of Application

To evaluate the effectiveness of the odour adsorbent filtering item, source sampling was prepared based on NCh 3386 and Dynamic Olfactometry Analysis according to NCh 3190:2010, which allows the measurement of odour concentration and approves the international standard EN 13725:2004.

Sixteen samples were taken where 8 samples were compared without considering a filtering item, 6 samples considering the multilayer adsorbent filtering item of the present invention and two samples with the filtering item in the state prior to the present invention.

An analysis of odour intensity (dynamic olfactometry) in the air samples was considered, considering the multilayer adsorbent filtering item or not, as the case may be. It is stated that "pig smell" is characterised by a high concentration of ammonia (NH₃), hydrogen sulphide (H₂S) and other volatile organic compounds (VOCs).

The evaluation was carried out in the olfactometry laboratory of Ecometrika (an internationally certified company) in Santiago. The tests were carried out according to the international protocols in force and using the "European Odour Unit (ou_{E}/m³)" as measurement standard. It is important to point out that the detection threshold of an odour is recorded at 5 ou_{E}/m³.

For this purpose, a total of 16 recirculation air samples were collected from pig houses, testing liquid and solid slurry for their very different odour load. Liquid slurry represents tests 1 to 4, while solid slurry is collected in tests 5 to 16.

### Odour Measurement Results

Table 1 shows the results obtained from tests performed in pig breeding centres for liquid slurry.

| Test number | Slurry type | Filtering element | Odour concentration (OU_{E}) | % of change in concentration |
|---|---|---|---|---|
| Test 1 | Liquid | Without filt. item | 96.835 | 97.4% |
| Test 2 | Liquid | With abs. fil. item | 2.545 | |
| Test 3 | Liquid | Without filt. item | 122.004 | 99.2% |
| Test 4 | Liquid | With abs. fil. item | 939 | |
| | | | | 98.3% |

According to the odour intensity test described in i), the effect of the adsorbent filtering item has a great impact generating a decrease in the concentration of the odour of 98.3% on average. It was observed that the reduction in European Odour Units reached 121.065 OU_{E} in the best of the cases seen, with and without the adsorbent filtering item of the invention.

The results of the tests on solid slurry with and without adsorbent filtering item of the invention are presented in table 2.

It was observed that solid slurry has a much less offensive odour emission than liquid slurry. The result of these tests showed a decrease in odour concentration of 90.3% on average.

| Test number | Slurry type | Filtering element | Odour concentration (OU_{E}) | % of change in concentration |
|---|---|---|---|---|
| Test 5 | Solid | Without filt. item | 447 | 95.3% |
| Test 6 | Solid | With abs. fil. item | 21 | |
| Test 7 | Solid | Without filt. item | 489 | 86.9% |
| Test 8 | Solid | With abs. fil. item | 64 | |
| Test 9 | Solid | Without filt. item | 512 | 90.4% |
| Test 10 | Solid | With abs. fil. item | 49 | |
| Test 11 | Solid | Without filt. item | 256 | 88.7% |
| Test 12 | Solid | With abs. fil. item | 29 | |
| | | | | 90.3% |

It can be observed that there are differences in the efficiency of the total concentration values, between the tests with and without the adsorbent filtering item (tests 1 to 12), where it is possible to obtain an average in the decrease of the total concentration greater than 93%, (see % of change in concentrations).

The results of tests 13 to 16 are depicted below, samples testing the filtering item prior to the invention (untreated activated carbon layer), where the average value in the decrease of the total concentration of the gases of 42% is observed, far below 93% of efficiency when the surface of activated carbon is modified, according to the present invention.

| Test number | Slurry type | Filtering element | Odour concentration (OU_{E}) | % of change in concentration |
|---|---|---|---|---|
| Test 13 | Solid | With prior filt. item | 117 | 49.6% |
| Test 14 | Solid | With abs. fil. item | 59 | |
| Test 15 | Solid | With prior filt. item | 140 | 35.0% |
| Test 16 | Solid | With abs. fil. item | 91 | |
| | | | | 42.3% |

It is concluded that the results of these tests prove that the multilayer adsorbent filtering item of the present invention solves the problem of offensive odours produced by pig breeding facilities with a greater efficiency in odour adsorption, with an unexpectedly surprising effect in comparison to a traditional porous adsorbent system.

Likewise, the quick action of the adsorbent filtering item on the pig odour compounds is surprising, since it requires a very short contact time to achieve its functional action.

To better understand the invention, it will be described based on the merely illustrative figures, the scope of the invention not being limited to the dimensions or to the quantity of illustrated elements.

### Brief Description of the Figures

Figure 1 shows the house where the multilayer adsorbent filtering item is tested.
Figure 2 shows the pore of a porous activated carbon support prior to the invention (layer 1).
Figure 3 shows part of the layers of the multilayer adsorbent filtering item of the present invention with the silicone film (layer 2).
Figure 4 shows the absorbent item of the present invention with the permanganate salt on silicone film (layer 3) .
Figure 5 shows the molecular lamination method for obtaining the multilayer adsorbent filtering item used in the example of application.

### Detailed Description of the Invention

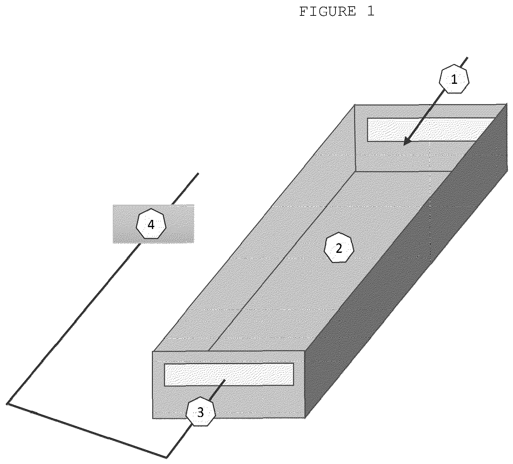

Figure 1 considers a house with an inlet (1) for air from the environment, a middle zone that considers the air inside the house (2), and an outlet for odorous gases that are channelled through ducts (3), to be treated (filtered).

The odorous gases are captured at the outlet of the ducts, either treated considered adsorbent filtering item (4) or untreated, depending on what is required for the tests. Finally, the gases are returned to the environment.

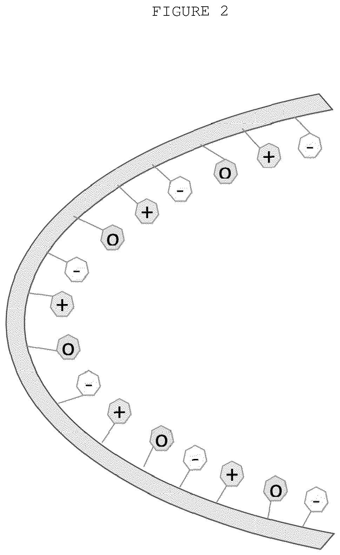

Figure 2 shows an adsorbent filtering item, which corresponds to layer 1, where the diagram depicts the view of a cross-section of the surface of the pore of the activated carbon and shows the different zones with polarities and/or positive charges (+), negative charges (-), and non-polar (0) .

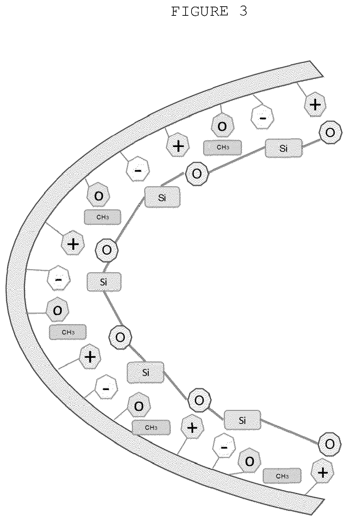

Figure 3 shows details of the multilayer adsorbent filtering item of the present invention with the silicone film, corresponding to layer 2. Silicone is an inorganic polymer derived from polysiloxane which is made up of a series of alternating silicon and oxygen atoms. It can be observed that the chemical structure of silicone, such as dimethylpolysiloxane, forms a chain starting with a silica atom (Si) bonded, on one side, to two methyl (CH₃) groups, and on the other end to 1 oxygen (O) atom, and so on so forth to form a chain.

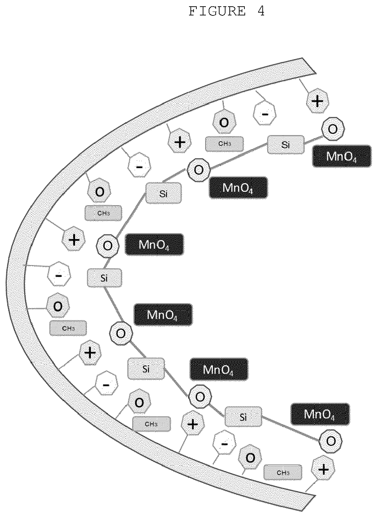

Figure 4 shows the multilayer absorbent filtering item of the present invention with the permanganate salt on silicone film (corresponding to layer 3). The interaction of the manganese (Mn) atom with the exposed oxygen (O) atoms of the silicone chain, dimethylpolysiloxane, allows anchoring and thus the third active surface of permanganate salt is formed.

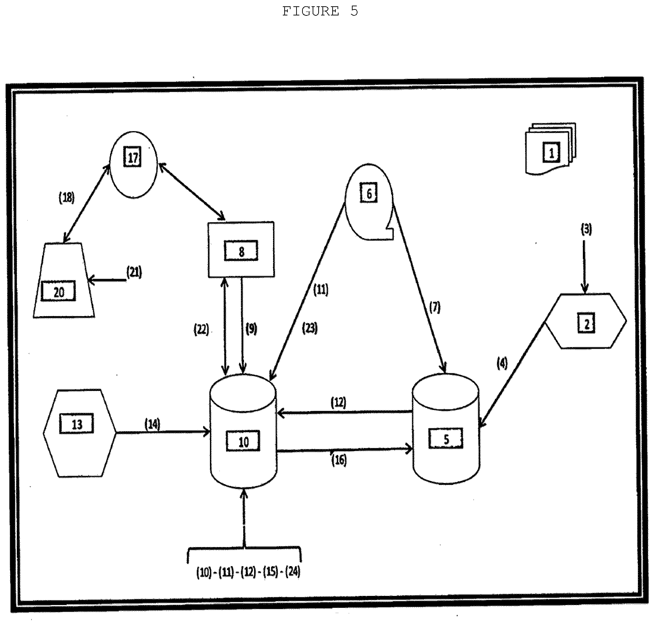

Figure 5 shows a flowchart of the system, which together with the flow and method lines show a procedure to achieve a high performance molecular lamination and to obtain the multilayer adsorbent filtering item, which will be detailed next.

The present invention comprises the multilayer adsorbent filtering item and uses a porous support consisting of a non-woven polyethylene terephthalate (PET) fibre felt impregnated with 40% activated vegetable carbon, having a specific surface area of at least 800 m²/g of carbon and a pore volume of at least 0.15 m³/g of carbon.

In the state of the art, it has been proposed to solve the problem of non-selective reactivity of the surface of the activated carbon by using mineral oil to covering the surface. This reactivity to be controlled is due to the functional groups such as hydroxyl, carbonyl, carboxyl, among others, which give it the amphoteric character, which can be acidic, positive or basic polarity, negative polarity (see Figure 2).

However, mineral oil has several drawbacks; first, it is hydrophobic, so it does not interact with the polar functional groups on the surface of the activated carbon; second, its viscosity and high surface tension prevents it from entering the pore and only manages to cover its external surface, eliminating the main property of the porous support, which is its large available surface area; and finally, ecological problem, because mineral oil is a non-biodegradable compound.

To solve these drawbacks, in the present invention, a coating film, such as a silicone film (see Figure 3), was applied.

For the present invention, it was determined that the compound to be used for coating the porous surface of the activated carbon and for producing the second layer of the multilayer adsorbent filtering item, should be an inert and biodegradable polymer such as liquid silicone, specifically dimethylpolysiloxane.

As mentioned above, the molecular structure of liquid silicone comprises methyl groups (neutral charge) and an oxygen group (polar charge), both bonded by silica atoms (an electrophile), which enables them to form a chain. This configuration allows non-polar bonds to form in the hydrophobic zones of the surface of the activated carbon using the methyl groups. On the other hand, when positively charged functional groups are present on the surface of the activated carbon, they interact through polar bonds with the oxygen group of the silicone. Finally, the negatively charged functional groups present on the surface of the activated carbon interact with the silica atom due to their electrophilic character by the displacement of the electron cloud by the oxygen molecule. All these interactions contribute to generate a "silicon film" that covers the functional groups present in the pore of the activated carbon and, consequently, prevent direct contact with the active ingredient, such as a permanganate salt

To obtain this second layer of the multilayer adsorbent filtering item, an aqueous mixture (mixture A) is prepared, which in the present invention is contacted with the porous activated carbon support as will be described in detail below, in such a way as to achieve a high performance molecular lamination.

In a first step, a silicone film is obtained which, when adhered to the surface of the activated carbon by means of apolar bonds (methyl group), on the opposite face, leaves the oxygen group available or exposed. The oxygen group (negative polarity) interacts with the manganese atom (positive polarity) and thus, the permanganate salt anchors to the silicone (see Figure 4).

To obtain this third layer of the multilayer adsorbent filtering item, an aqueous mixture (mixture B) is prepared, which in the present invention is contacted with the porous activated carbon support containing the second silicone layer, as explained in the method for achieving a high performance molecular lamination.

In this second step, a surface covered with the permanganate salt (MnO₄⁻) is obtained, with its active site (-MnO⁻) available to the medium, that is, a functional product with oxidative properties to act on the compounds (chemical structures and VOCs) responsible for the offensive odours produced by bred animals such as pigs and birds.

The method for obtaining the multilayer absorbent filtering item comprises three steps: coating the porous support corresponding to activated carbon (first layer) with a mixture (A), containing water, silicone (dimethylpolysiloxane), and low-foam surfactants (ethoxylated alcohols), constituting the second layer; followed by coating with the mixture (B) prepared with potassium permanganate salt and low-foam surfactant (ethoxylated alcohol) in an acidic aqueous medium, thereby forming a third layer. Finally, a drying method is carried out by eliminating the excess water contained in the pores and thereby obtaining the product of the present invention, the multilayer adsorbent filtering item.

Although a method for performing impregnation under vacuum conditions like in the present invention is described in the state of the art (with some adaptations), the purpose has been to seal surfaces of items which are exposed to extreme conditions, and this method has not been applied, as described in the present invention, on surfaces having a high porosity and large surface area, with the aim of creating a multilayer adsorbent filtering item having a large surface area and great functionality.

The method flows used are described below (Figure 5).

To begin with, the molecular lamination method for the multilayer adsorbent filtering item of the present invention, mixtures (A) and (B) are first prepared.

As shown in Figure 5, the aqueous mixture A (3) is prepared in the mixing tank (2). The mixture of the present invention has the advantage over the state of the art of other mixtures because it contains a low-foam surfactant agent (ethoxylated alcohol) allowing the surface tension of the mixture to be lowered, facilitating and enabling the penetration of the solutes into the pores of the activated carbon. In this way, the silicone molecules contained in the mixture (A) are uniformly distributed on the surface of the pore walls of the activated carbon, forming the second layer.

To determine a coverage value with mixture A, in practice, the chemical was considered at a low concentration (less than 10% w/v) and of surfactant (less than 2% w/v) by weight/volume of the total mixture.

Mixture A is transferred (4) to the degassing tank (5), where a vacuum of less than 10,000 Pa is applied for 5 to 10 minutes by means of a vacuum pump (6), in order to produce a degassing (7), preventing the gases dissolved in the mixture from interfering with the breaking of the surface tension in the molecular lamination step of the pores of the activated carbon.

At the same time, the porous support adsorbent filtering multilayer item prior to the invention, contained in a basket (8) to control the buoyancy, is introduced in the autoclave (10), where a vacuum (11) lower than 200 Pa is applied for a period of between 20 and 30 minutes, giving the porous support the distinctive property of not containing air in the pores. A vacuum pump (6) is used to achieve this vacuum level.

The next step is started by transferring (12) mixture A from the degassing tank (5) to the autoclave (10), with the essential characteristic that this filling must be done in an upward manner. Likewise, during the entire upward filling process (12) of the autoclave (10), it is a fundamental condition to maintain a vacuum level below 500 Pa, since only in this way is it possible to quickly access the interior of the pores of the activated carbon while these are free of oxygen.

In this way, it is ensured that the molecular lamination, which consists of the molecules being uniformly distributed throughout the porous support, constitutes a homogeneous and consistent layer over time. Then, the adsorbent filtering item is kept submerged and under vacuum for a period of 20 to 30 minutes.

Afterwards, the vacuum is broken (15) and the autoclave (10) is unloaded, transferring the mixture (16) back to the degassing tank (5), using the remaining vacuum of the degassing tank. Once the autoclave (10) is emptied, the machinery is cleaned.

In the next step of the method, the basket (8) with the adsorbent filtering item inside is removed from the autoclave (10) and transferred (18) to a centrifuge (20). A key (17) is used for this function. Then a centrifugation step (21) is applied between 200 and 1000 rpm for a period of 1 to 6 minutes at a gradually increasing speed, to eliminate the excess mixture.

Finally, the adsorbent filtering item with the second layer, already centrifuged, is transferred (22) again to the autoclave (10), where it is subjected to a vacuum below 500 Pa (23) and a temperature between 30°C and 36°C (24).

The combination of both factors, vacuum and temperature, allows a rapid evaporation of water at low temperature, achieving that the silicone (dimethylpolysiloxane) is deposited on the porous surface and forms a uniform layer. This corresponds to the last step of the molecular lamination method of the second layer, remaining only to remove the basket (8) with the porous support contained therein using the key (17).

In this way, an adsorbent filtering item containing silicone is obtained (the second layer), ready to continue with the method to add the third layer (permanganate salt).

For the second step, mixture B (3) is prepared in the mixing tank (2). The mixture contains a low-foam surfactant agent (ethoxylated alcohol) allowing the surface tension of the mixture to be lowered, facilitating the penetration of the solutes into the pores, while the presence of an acidic medium allows the stability of the permanganate salt. In this way, the mixture is uniformly distributed on the surface of the pore walls of the activated carbon.

To determine a coverage value with the solvent, in practice, the permanganate salt was considered at a low concentration (less than 6% w/v) and of a surfactant (less than 1% w/v) by weight/volume of the total mixture.

Mixture B is transferred (4) to the degassing tank (5), where a vacuum of more than 10,000 Pa is applied for 5 to 10 minutes by means of a vacuum pump (6), in order to produce a degassing (7), preventing the gases dissolved in the mixture from interfering with the breaking of the surface tension in the molecular lamination step of the pores of the activated carbon.

At the same time, the adsorbent filtering item with the silicone layer, contained in a basket (8) to control the buoyancy, is introduced (9) in the autoclave (10), where a vacuum (11) below 200 Pa is applied for a period of between 20 and 30 minutes, giving the adsorbent filtering item with the silicone layer the distinctive property of not containing air in the pores. A vacuum pump (6) is used to achieve this vacuum level.

The next step is started by transferring (12) the mixture from the degassing tank (5) to the autoclave (10), with the essential characteristic that this filling must be done in an upward way. Likewise, during the entire upward filling method (12) of the autoclave (10), it is a fundamental condition to maintain a vacuum level below 500 Pa, since only in this way is it possible to quickly access the interior of the pores of the activated carbon while these are free of oxygen. In this way, it is ensured that the molecular lamination, which consists of the molecules being uniformly distributed throughout the adsorbent filtering item with the silicone layer, constitutes a homogenous and consistent layer in the permanganate salt time. The adsorbent filtering item with the silicone and permanganate salt layers is kept submerged and under vacuum for a period of between 20 and 30 minutes.

Afterwards, the vacuum is broken (15) and the autoclave (10) is unloaded, transferring the mixture (16) back to the degassing tank (5), taking advantage of the remaining vacuum in the degassing tank. Once the autoclave is emptied (10), the machinery is cleaned.

In the next step of the method, the basket (8) with the adsorbent filtering item with the silicone and permanganate salt layers contained therein is removed from the autoclave (10) and transferred (18) to a centrifuge (20). A key (17) is used for this function. Then a centrifugation step (21) is applied between 200 and 1000 rpm for a period of 1 to 6 minutes of gradual speed, to eliminate the excess mixture.

Finally, the adsorbent filtering item with the silicone and permanganate salt layers, already centrifuged, is transferred (22) again to the autoclave (10), where it is subjected to a vacuum below 500 Pa (23) and a temperature between 30°C and 36°C (24).

The combination of both factors, vacuum and temperature, allows a rapid evaporation of water at low temperature, achieving that the permanganate salt is deposited on the porous surface and forms a uniform layer. This corresponds to the last step of the molecular lamination method, remaining only to remove the basket (8) with the porous support contained therein using the key (17).

In the manner described herein, the multilayer adsorbent filtering item of the present invention is finally obtained to eliminate offensive odours from bred farm animals, such as pigs and birds.

In order to determine the content of solids incorporated into the pores, the dry weight was measured by gravimetry and infrared radiation until a constant moisture-free mass was obtained.

In this way, the practice was developed and through a mass balance, the value of added mass of the multilayer adsorbent filtering item is obtained, where according to the experience previously described, values comprised between 500 and 120% of added mass of silicone (second layer) by weight/dry, and between 200% and 300% of added mass of permanganate salt (third layer) by weight/dry, with respect to the weight of the activated carbon, were obtained.

## Claims

1. Method for obtaining a multilayer adsorbent filtering item, the method comprising:
- coating a porous activated carbon support disposed on a non-woven polyester fibre felt, constituting a first layer with a mixture (A), wherein the mixture (A) contains water, a liquid silicone polymer, and low-foam surfactants, constituting a second layer; next,
- coating the second layer with a mixture (B) prepared from potassium permanganate salt and low-foam surfactant in an acidic aqueous medium, thereby forming a third layer; and finally,
- drying such that a multilayer adsorbent filtering item is obtained, wherein the liquid silicone polymer is dimethylpolysiloxane and the low-foam surfactants are ethoxylated alcohols.

2. A multilayer adsorbent filtering item obtainable according to claim 1.

3. The multilayer adsorbent filtering item according to claim 2, **characterised in that** the non-woven polyester fibre felt is a non-woven polyethylene terephthalate fibre felt.

4. Use of the multilayer adsorbent filtering item according to claim 2 to eliminate offensive odours from bred farm animals.

5. Use of the multilayer adsorbent filtering item according to claim 4 to eliminate offensive odours from pigs and birds.

## Patentansprüche

1. Verfahren zum Erhalten eines mehrschichtigen adsorbierenden Filterartikels, wobei das Verfahren Folgendes umfasst:
- das Beschichten eines porösen Aktivkohle-Träger, welcher auf einem nichtgewebten Polyesterfase-Filz angeordnet ist, unter Bildung einer ersten Schicht mit einer Mischung (A), wobei die Mischung (A) Wasser, ein flüssiges Silikonpolymer und schwachschäumende Tenside enthält, unter Bildung einer zweiten Schicht; anschließend,
- das Beschichten der zweiten Schicht mit einer Mischung (B), welche aus Kaliumpermanganat-Salz und schwachschäumendem Tensid in einem sauren wässrigen Medium vorbereitet wird, wobei dadurch eine dritte Schicht gebildet wird; und schließlich,
- das Trocknen, sodass ein mehrschichtiger adsorbierender Filterartikel erhalten wird, wobei das flüssige Silikonpolymer Dimethylpolysiloxan ist, und die schwachschäumenden Tenside ethoxylierte Alkohole sind.

2. Mehrschichtiger adsorbierender Filterartikel, welcher nach Anspruch 1 erhalten werden kann.

3. Mehrschichtiger adsorbierender Filterartikel nach Anspruch 2, **dadurch gekennzeichnet, dass** der nichtgewebte Polyesterfase-Filz ein nichtgewebter Polyethylenterephthalatfaser-Filz ist.

4. Verwendung des mehrschichtigen adsorbierenden Filterartikels nach Anspruch 2, um üble Gerüche aus gezüchteten landwirtschaftlichen Nutztieren zu beseitigen.

5. Verwendung des mehrschichtigen adsorbierenden Filterartikels nach Anspruch 4, um üble Gerüche aus Schweinen und Vögeln zu beseitigen.

## Revendications

1. Procédé pour obtenir un article filtrant adsorbant multicouche, le procédé comprenant :
- enduire un support poreux de charbon actif disposé sur un feutre de fibre de polyester non tissée, constituant une première couche avec un mélange (A), dans lequel le mélange (A) contient de l'eau, un polymère de silicone liquide et un surfactant peu moussant, constituant une deuxième couche ; puis,
- enduire la deuxième couche avec un mélange (B) préparé à partir de sel de permanganate de potassium et de surfactant peu moussant dans un milieu aqueux acide, formant ainsi une troisième couche ; et finalement,
- sécher de telle sorte qu'un article filtrant adsorbant multicouche est obtenu, dans lequel le polymère de silicone liquide est du diméthylpolysiloxane et les surfactants peu moussants sont des alcools éthoxylés.

2. Article filtrant adsorbant multicouche obtenable selon la revendication 1.

3. Article filtrant adsorbant multicouche selon la revendication 2, **caractérisé en ce que** le feutre de fibre de polyester non tissée est un feutre de fibre de polyéthylène téréphtalate non tissée.

4. Utilisation de l'article filtrant adsorbant multicouche selon la revendication 2, pour éliminer les mauvaises odeurs provenant d'animaux d'élevage en ferme.

5. Utilisation de l'article filtrant adsorbant multicouche selon la revendication 4 pour éliminer les mauvaises odeurs provenant de porcs et d'oiseaux.
